# EUROPÄISCHE PATENTANMELDUNG

(11) **EP 1 234 561 A2**
(43) Veröffentlichungstag der Anmeldung: **28.08.2002**
(21) Anmeldenummer: 02007539.6
(22) Anmeldetag: 22.10.1997
(51) Int. Cl.: A61F 9/00

(54) **Verfahren zur Formgebung von Oberflächen, insbesondere von Linsen**

(30) Priorität: 26.10.1996 DE 19644664; 28.06.1997 DE 19727573
(62) Teilanmeldung aus: 97945865.0
(71) Anmelder: Asclepion Meditec AG, 07745 Jena (DE)
(72) Erfinder: Kühnert, Jürgen, 07747 Jena (DE); Mäusezahl, Holger, 07745 Jena (DE); Pieger, Stefan, 90530 Wendelstein (DE); Schröder, Eckhard, 90542 Eckental (DE)
(74) Vertreter: Pfeiffer, Rolf-Gerd, Dipl.-Phys.

(57) **Zusammenfassung**

Die Erfindung betrifft ein Verfahren zur Formgebung von Oberflächen, insbesondere von Linsen, vermittels einer Laserabtragung der Oberflächen. Bevorzugt findet die Erfindung Anwendung bei der Photo Refraktiven Keratektomie und ophthalmologisch exakten Formung von Kontaktlinsen. Die Aufgabe der Erfindung, eine Lösung anzugeben, die gewährleistet, daß bei einem plötzlichen Oberflächenbearbeitungsabbruch wenigstens eine akzeptable Teilkorrektur der zu bearbeitenden Oberfläche vorliegt, wird dadurch gelöst, daß die Vorrichtung einen zum Laserscanning eingesetzten gepulsten Laser (1), eine Strahlformungseinheit (2) und eine Laserstrahlablenkeinheit (3), die den Laserstrahl auf eine zu bearbeitende Oberfläche (4) ablenkt enhält, wobei die Laserstrahlablenkeinheit (3) und die Strahlformungseinheit (2) von einer Ansteuereinheit (5) durch das Zusammenwirken einer ersten (6) und zweiten Eingabeeinheit (7), einer Berechnungseinheit (8), der die Daten der ersten und zweiten Eingabeeinheiten (6; 7) zugeführt werden, eines Blockgenerators (9), dem die in der Berechnungseinheit (8) gewonnen Daten zugeführt werden und in dem die Einzellaserschußkoordinaten zur Abtragung jeweils einer beliebig vorgebbaren abzutragenden Einzeloberflächenschicht einander zugeordnet werden, und eines Ablaufgenerators (10), an den die, einem Einzelabtragungsgebiet entsprechenden, in Blöcken zusammengefaßten, vom Blockgenerator (9) erzeugten Daten weitergeleitet werden und in dem die einzelnen Blöcke zusammengefaßt und in eine vorgebbare Reihenfolge gebracht werden, derart ansteuerbar sind, daß eine vom Ablaufgenerator (10) gesandte bestimmte Blocksequenz auf die Laserstrahlablenkeinheit (3) und auf eine Strahlformungseinheit (2) in der Weise einwirken, daß pro Blocksequenz jeweils eine vollständige Teilkorrektur der zu bearbeitenden Oberfläche (4) gewährleistet ist und weiterhin eine Zentralrechnereinheit (11) vorgesehen ist, die in Abhängigkeit von den die Ansteuereinheit (5) verlassenden Ansteuerdaten die Zuschaltung und Steuerung zumindest der Lasereinheit (1), der Strahlformungseinheit (2; 21) und der Laserstrahlablenkeinheit (3) in vorgebbare Weise koordiniert.

## Beschreibung

Die Erfindung betrifft ein Verfahren zur Formgebung von Oberflächen, insbesondere von Linsen, vermittels einer Laserabtragung der Oberflächen, insbesondere, jedoch nicht ausschließlich, von Oberflächen biologischer Materialien. Bevorzugt findet die Erfindung Anwendung bei der Photo Refraktiven Keratektomie (PRK) und ophthalmologisch exakten Formung von Kontaktlinsen.

Der bekannte Stand der Technik soll im nachstehenden anhand des dem der Erfindung am nächsten kommenden beschrieben werden, der sich mit der Korrektur von Fehlsichtigkeit befaßt.
Die seit Mitte der 80er Jahre durchgeführten ablativen Verfahren zur Behandlung von Fehlsichtigkeiten am menschlichen Auge beruhen alle auf den erstmalig von Munnerlin beschriebenen Formeln für die Berechnung der nötigen Abflachung bzw. Aufsteilung der Hornhaut. Im Falle der Korrektur einer Kurzsichtigkeit wird gemäß dieser Berechnungen mehr Hornhautgewebe im Hornhautzentrum als im peripheren Bereich der Hornhaut entfernt. Im Falle der Korrektur von Weitsichtigkeit wird mehr Gewebe in der Hornhautperipherie als im Zentrum der Hornhaut abgetragen. Der dadurch auf die Brechkraft der Hornhautoberfläche entstehende verstärkende oder abschwächende Effekt entspricht in seiner Wirkung dem einer Kontaktlinse.

Die Entfernung der entsprechenden Hornhautmenge erfolgt bei allen bekannten, der Erfindung nahekommenden Verfahren in einzelnen, aufeinanderfolgenden Bereichen von sich stetig verändernden Arealen. Im theoretisch einfachsten Fall, einer Korrektur sphärischer Myopie, werden diese Areale durch aufeinanderfolgende Kreise mit stetig kleiner oder größer werdenden Durchmessern beschrieben. Für die Erzielung eines optimalen Behandlungsergebnisses ist es notwendig, alle Schichten einer Serie komplett abzuarbeiten.

Kennzeichnend für die derzeitig bekannten Verfahren ist, daß diese Serien immer vom Beginn bis zum Ende in stetig steigender oder fallender Reihenfolge abgearbeitet werden. Kommt es bei diesen Verfahren zu einem ungewollten Behandlungsabbruch, entstehen unregelmäßige Hornhautoberflächen, die in der Regel zu einer deutlichen Verschlechterung des Sehvermögens führen. Leider lassen sich so verursachte Sehfehler mit konventionellen Mitteln, wie einer Brille oder Kontaktlinse, nicht oder nur unzureichend beheben. Einziger Ausweg ist es, die Behandlung an genau dem Punkt fortzusetzten, an dem sie unterbrochen wurde. Kann der genaue Punkt der Behandlungsunterbrechung nicht festgestellt werden oder ist der Patient nicht bereit, eine weitere Behandlung über sich ergehen zu lassen, kommt es zu einer dauernden Verschlechterung des Sehvermögens.
Eine umfassende Beschreibung des bislang bekannten Standes der Technik ist in EP 90 308 709.6 (entspricht DE 690 24 558 T2) abgehandelt.
In US-PS 5,520,679 ist eine ophthalmologische Operationsmethode unter Verwendung eines Spot Scanning Lasers beschrieben, bei der der Hornhautabtrag durch Setzen einzelner Laserspots erfolgt. Bei diesem Verfahren, das im übrigen wie die o.g. Abtragverfahren durchgeführt wird, wird versucht, einen flächenmäßig möglichst gleichmäßigen Abtrag dadurch zu erzielen, daß zeitlich unmittelbar aufeinanderfolgende Laserspots mit einem definierten Überlappungsverhältnis gesetzt werden. Dies bedingt jedoch, insbesondere bei Einsatz eines Erbium-Lasers eine erhöhte thermische Belastung einzelner Flächenareale.

Ein ähnliches Verfahren ist in WO 96/11655 beschrieben. Dort werden Schußpositionen nach einem empirischen Algorithmus auf Ringen mit einem bestimmten Radius angeordnet. Auf jedem Ring sind dabei eine Vielzahl sich relativ weit überlappender Schüsse vorgesehen. Insbesondere bei der Bearbeitung der inneren Ringe kann es, aufgrund der starken Überlappung der einzelnen Schußpositionen, möglich sein, die Schußreihenfolge so zu setzen, daß eine lokale Überhitzung vermieden wird. Zur Vermeidung der lokalen Überhitzung wird die Schußreihenfolge deshalb nach WO 96/11655 so fest gelegt, daß sich nacheinander folgende Schüsse nicht überlappen. In einer speziellen Ausführung werden die Schüsse so sortiert, das der jeweilige Abstand zwischen ihnen maximal ist. Weiterhin wird dort vorgeschlagen, das Schußfeld nach dem Zufallsprinzip neu zu ordnen, damit statistisch die Zahl sich überlappender Schüsse reduziert wird. Einziges Kriterium für das zufällige Setzen der einzelnen Schüsse nach WO 96/11655 ist somit, die Vermeidung der lokalen Überhitzung.
Entsprechend der nach WO 96/11655 gesetzten Einzelspots, die auf vorgegebenen Bahnen (rings) derart gesetzt werden, daß sich Einzelspots eines Laserstrahls mit großem Durchmesser überlappen, ist eine vollständige Teilkorrektur nicht erreichbar. Nach WO 96/11655 wird bei der Myopie mit dem kleinsten Ring gestartet und der Durchmesser des Abtraggebietes anschließend kontinuierlich erhöht. Sollte es hierbei zu einem Abbruch (bspw. Stromausfall) kommen, ist nur das Zentrum bearbeitet, wobei ein unbestimmter Zustand, ein optisches Chaos, der u.U. zu einer Verschlechterung des Ausgangszustandes geführt haben kann, erreicht wurde. Gleiches trifft bei der Hyperopie zu, wo der Abtragprozeß kontinuierlich von außen beginnend erfolgt.

Weiterhin ist in EP 0 247 260 A2 eine Methode und eine Vorrichtung zur Radialen Keratotomie beschrieben. Bei der Radialen Keratotomie werden auf der Hornhaut Schnitte angebracht; die Abflachung der Hornhaut resultiert mittelbar aus der limbusparallelen Dehiszenz der radiär orientierten Wundränder. Die Aufgabe von EP 0 247 260 A2 besteht darin, die Wirkung dieser chirurgischen Eingriffe vorhersehbarer zu machen. Zu diesem Zweck wird dort eine Erfahrungsdatenbank aufgebaut, in der die Werte vor und nach einer vollständig durchgeführten Operation sowie die für diese Operation durchgeführten medizinischen Maßnahmen gespeichert werden. Ferner ist in dieser Schrift ausgeführt, daß auch bei der photorefraktiven Keratotomie die Vorhersehbarkeit des Behandlungsergebnisses einer vollständig durchgeführten Operation noch nicht ausreichend ist, so daß auch hierfür der Aufbau einer analogen Erfahrungsdatenbank vorgeschlagen wird. In jedem Fall wird nach EP 0 247 260 A2 also der Endzustand von verschiedenen, zeitlich weit auseinanderliegenden Operationen erfaßt und das Endergebnis mit dem jeweiligen Ausgangszustand verglichen. Die in EP 0 247 260 A2 angegebene Erfahrungsdatenbank (für einen unerfahrenen Operateur) ist keine, die aktiv innerhalb eines einheitlichen Abtragungsprozesses eingesetzt werden kann oder dafür einsetzbar wäre.

Es wird dort, damit die Unerfahrenheit des Operateurs nicht zu einer Überkorrektur führt, lediglich in Betracht gezogen, eine Operation vorzeitig abzubrechen, um nach einer sich anschließenden Wundheilung mit einer zweiten Operation, ausgehend von dem nunmehr vorliegenden Zustand und Vergleich mit eventuell gleichgelagerten Ausgangsfällen und den dafür vorgesehenen Maßnahmen in der Erfahrungsdatenbank, ggf. zu einem dem gewünschten Endzustand besseren Ergebnis zu gelangen.

Der Erfindung liegt die Aufgabe zugrunde, ein Verfahren zur Formgebung von Oberflächen, insbesondere von Linsen und hierbei insbesondere von Oberflächen biologischer Materialien unter Verwendung eines Lasers mit gepulstem Laserausgangsstrahl anzugeben, die gewährleisten, daß bei einem plötzlichen gewollten oder ungewollten Oberflächenbearbeitungsabbruch wenigstens eine akzeptable Teilkorrektur der zu bearbeitenden Oberfläche vorliegt, die einer Verbesserung der Verhältnisse des Ausgangszustandes darstellt. Weiterhin ist es Aufgabe der Erfindung, die thermische Belastung, insbesondere bei Verwendung eines Erbium-Lasers, der zu bearbeitenden Oberfläche weitestgehend zu reduzieren.
Die Aufgabe wird durch die kennzeichnenden Merkmale von Patentanspruch 1 gelöst. Vorteilhafte weitere Ausgestaltungen sind in den nachgeordneten Ansprüchen erfaßt.

Bei dem hier vorgestellten Verfahren werden die einzelnen Schichten einer zu bearbeitenden Oberfläche in einer vorbestimmten Reihenfolge abgetragen, um das Risiko einer Verschlechterung des Sehvermögens bei unvorhergesehenen Behandlungsabbrüchen zu minimieren. Anstelle einer Bearbeitung, bei der erst am Ende eine optisch einwandfreie, sphärische Hornhautoberfläche mit der gesamten, angestrebten Korrektur entsteht, werden beim vorgeschlagenen Verfahren möglichst viele Teilkorrekturen mit akzeptablen Zwischenergebnissen erzeugt. Bei einem plötzlichem Behandlungsabbruch kommt es deshalb nie zu stark unregelmäßigen bearbeiteten Oberflächen. In solch einem Fall wird zwar nicht die volle, durch die Bearbeitung angestrebte Korrektur erreicht, doch der verbleibende Sehfehler kann wesentlich einfacher mit einer Brille oder Kontaktlinse behoben werden. Darüber hinaus kann zu einem späteren Zeitpunkt eine erneute PRK wesentlich unkomplizierter, auch mit nach anderen Verfahren arbeitenden Geräten vorgenommen werden.

Das beschriebene Formgebungsverfahren eignet sich für alle derzeit verwendeten PRK-Methoden wie Area Ablation, Slit Scanning und Small Spot Scanning, sowohl bei Ablationen auf der Hornhautoberfläche als auch intrastromale Gewebeabtragungen in Verbindung mit der LASIK-Methode oder Pikosekunden Lasern.

Das vorgeschlagene Verfahren unterscheidet sich von der bei nach dem Area Ablation Prinzip arbeitenden Geräten gelegentlich verwendeten Multi Pass Methode dadurch, daß hier bewußt sehr viele, möglichst kleine Teilkorrekturen erzeugt werden. Beim Multi Pass erfolgt typischerweise nur eine Aufteilung in 2 - 3 Teilkorrekturen. Außerdem wird mit der Multi Pass Technik in erster Linie versucht, bestimmte für die Area Ablation typische Nebeneffekte, wie einer zu starken Austrocknung, einer zu starken Gewebeerwärmung und der Entstehung von Central Islands zu reduzieren.

Die Erfindung soll nachstehend anhand eines schematischen Ausführungsbeispiels näher erläutert werden. Es zeigen:
- Fig. 1: blockschaltbildartig die wesentlichen beim Verfahren zum Einsatz gelangenden Komponenten sowie ihre funktionelle Zuordnung zueinander,
- Fig. 2: beispielhaft die Abfolge erforderlicher Bearbeitungsschritte für eine Myopie Korrektur im Falle der Anwendung des sogenannten Small Spot Scanning nach dem Stand der Technik und
- Fig. 3: eine Möglichkeit der erfindungsgemäßen Abfolge erforderlicher Bearbeitungsschritte für eine Myopie Korrektur ebenfalls im Falle der Anwendung des Small Spot Scanning.

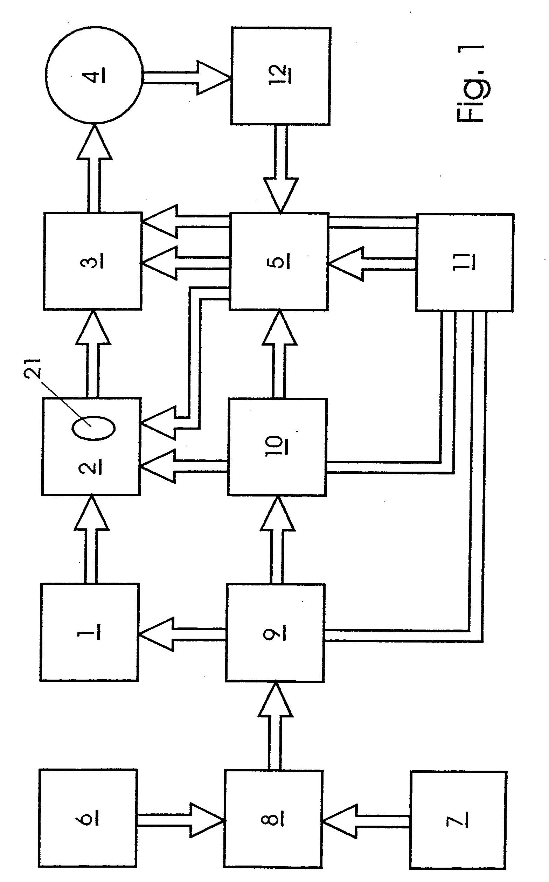

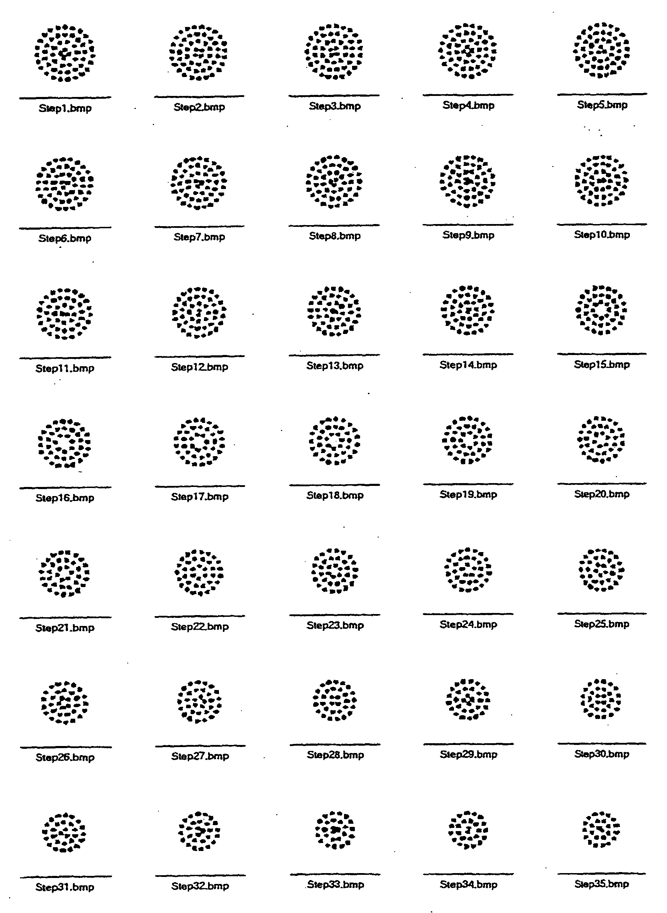

In Figur 1 sind blockschaltbildartig die wesentlichen Komponenten, die im Verfahren zum Einsatz gelangen dargestellt. Zunächst ist ein an sich bekannter, zum Laserscanning eingesetzter gepulster Laser 1 vorgesehen.

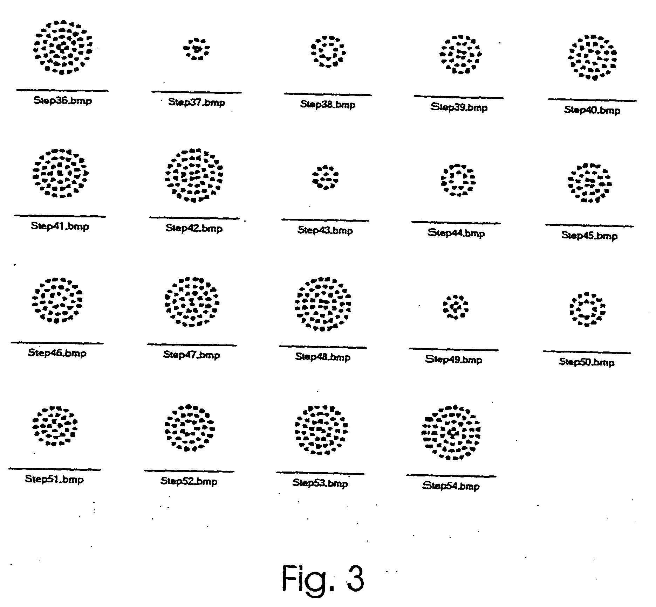

Für diesen Laser kommen Laser mit gepulstem Laserausgangsstrahl, bspw. UV-Laser, wie Eximerlaser, Er:YAG-Laser oder gütegeschaltetem Laser für den vorgesehenen Verwendungszweck geeigneter Wellenlänge und Intensität bzw. Energie in Betracht. Dem Laser 1 ist eine Strahlformungseinheit 2 nachgeordnet, die, wie nach dem Stand der Technik bekannt, aus Linsen, Spiegeln und Prismen gebildet ist. Weiterhin ist eine, auf die Laserimpulsfolge abgestimmte Laserstrahlablenkeinheit 3 vorgesehen, die die Laserstrahlung definiert auf die zu bearbeitende Oberfläche 4 ablenkt. Die Laserstrahlablenkeinheit 3 und die Strahlformungseinheit 2 sind mit einer Ansteuereinheit 5 in Verbindung gebracht. Zum definierten Ansteuern der Ansteuereinheit 5 ist zunächst eine erste Eingabeeinheit 6 vorgesehen, die die refraktiven Eingangsgrößen (z.B. dpt, sph, zyl) der zu bearbeitenden Oberfläche 4 aufnimmt. Diese Ausgangsparameter sind zunächst auf übliche Weise, unabhängig von der Bearbeitungsvorrichtung, zu ermitteln. Weiterhin ist eine zweite Eingabeeinheit 7 vorgesehen, welche vornehmlich zur Aufnahme laserrelevanter Daten dient. Solche Daten betreffen den Laserstrahldurchmesser, die Energiedichte des Laserstrahls und die Energieverteilung über den Laserstrahlquerschnitt und einen Überlappungsfaktor, welche einzeln als auch in ihrer Gesamtheit variabel vorgebbar sind. Diesen beiden Eingabeeinheiten 6, 7 ist eine Berechnungseinheit 8 zugeordnet, innerhalb derer, alle erforderlichen Lasereinzelschußkoordinaten in Abhängigkeit von der gesamt abzutragenden Oberfläche 4 einander zugeordnet werden. Das heißt, dort wird ermittelt und festgelegt, wieviele Laserspots in welchem Teilgebiet der abzutragenden Oberfläche in Abhängigkeit der Laserparameter zu setzen sind, um die Oberfläche 4 in den gewünschten Sollzustand zu überführen. Der Berechnungseinheit 8 ist ein sogenannter Blockgenerator 9 nachgeordnet, dem die Daten der Berechnungseinheit 8 zugeführt werden, und in dem die Einzellaserschußkoordinaten zur Abtragung jeweils einer beliebig vorgebbaren abzutragenden Einzeloberflächenschicht (vgl. bspw. Fig. 3 ein beliebiges, durch einen Step x erfaßtes Gebiet) in solcher Weise einander zugeordnet werden (in Blöcke zusammengefaßt werden), daß zeitlich unmittelbar aufeinanderfolgende Laserspots im Einzelabtragungsgebiet in der Folge voneinander so beabstandet gesetzt werden, daß sie keine Überlappung erfahren, was vorteilhaft über eine Zufallsverteilung erreicht wird und bei der Durchführung des Verfahrens den Vorteil nach sich zieht, daß die thermische Belastung im Abtragungsgebiet minimiert wird. Dem Blockgenerator 9 ist schließlich ein Ablaufgenerator 10 nachgeordnet, in dem die einzelnen, im Blockgenerator 9 gebildeten Blöcke zusammengefaßt und in eine der Erfindung zugrundeliegende Reihenfolge gebracht werden. Diese Reihenfolge stellt jeweils eine Blocksequenz dar, die anhand von Fig. 3 den Bearbeitungsteilschritten (Step1 bis 6; 7-12; 13-18; 19-24; 25-30; 31-36; 37- 42; 43-48; 49-54) entspricht. Jeder dieser Bearbeitungsteilschritte stellt dabei in sich eine vollständige Teilkorrektur dar. Die durch das Zusammenwirken bisher genannter Einheiten vom Ablaufgenerator 10 an die Ansteuereinheit 5 gesandte definierte Blocksequenz dient zum einen der Ansteuerung der Laserstrahlablenkeinheit 3 und zum anderen der Ansteuerung der Strahlformungseinheit 2 und dort insbesondere zur Ansteuerung einer in der Strahlformungseinheit 2 vorgesehenen, in ihrer Öffnung veränderbaren Blende 21, deren Funktion beim Ablauf des Verfahrens näher erläutert wird. Weiterhin ist eine Zentralrechnereinheit 11 vorgesehen, die in Abhängigkeit von den die Ansteuereinheit 5 verlassenden Ansteuerdaten die Zuschaltung und Steuerung zumindest der Lasereinheit 1, der Strahlformungseinheit 2; 21 und der Laserstrahlablenkeinheit 3 in vorgebbare Weise koordiniert.
Es liegt im Rahmen der Erfindung, die erste und zweite Eingabeeinheit 6; 7, die Berechnungseinheit 8, den Blockgenerator 9, den Ablaufgenerator 10 und die Ansteuereinheit 5 einer zweiten, mit dem Zentralrechner 11 in Verbindung stehenden Rechnereinheit zuzuordnen. Auch ist es möglich, die genannten Einheiten im Zentralrechner 11 zu integrieren.
Zusätzlich zu den bis hierher beschriebenen Einheiten ist vorteilhaft eine Überwachungseinheit 12 vorgesehen, die zufällige Auslenkbewegungen der zu bearbeitenden Oberfläche 4 erfaßt und über die Ansteuereinheit 5 entsprechende Korrektursignale an die Laserstrahlablenkeinheit 3 weiterleitet. Eine solche Überwachungseinheit ist z.B. von A. Unkroth et al. in "Corneal surgery by two-dimensionally scanning of a low-energy excimer laser beam" SPIE Vol. 2126 Ophthalmic Technol. IV 1994 S. 217ff. beschrieben.

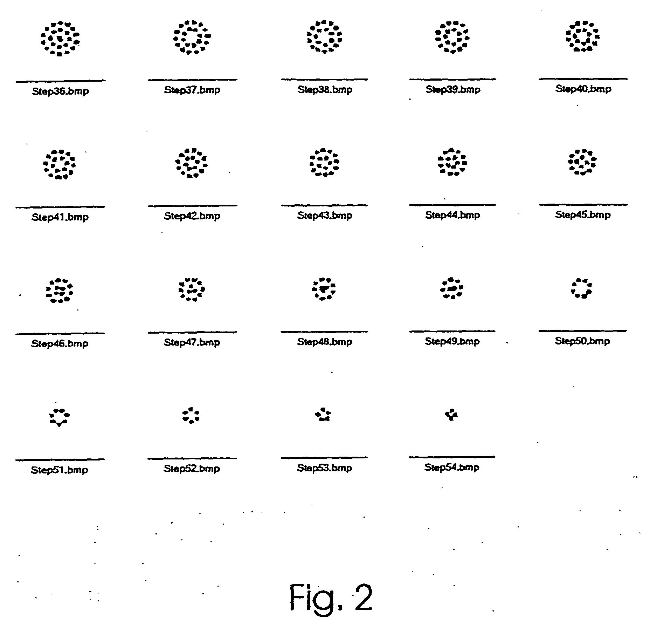

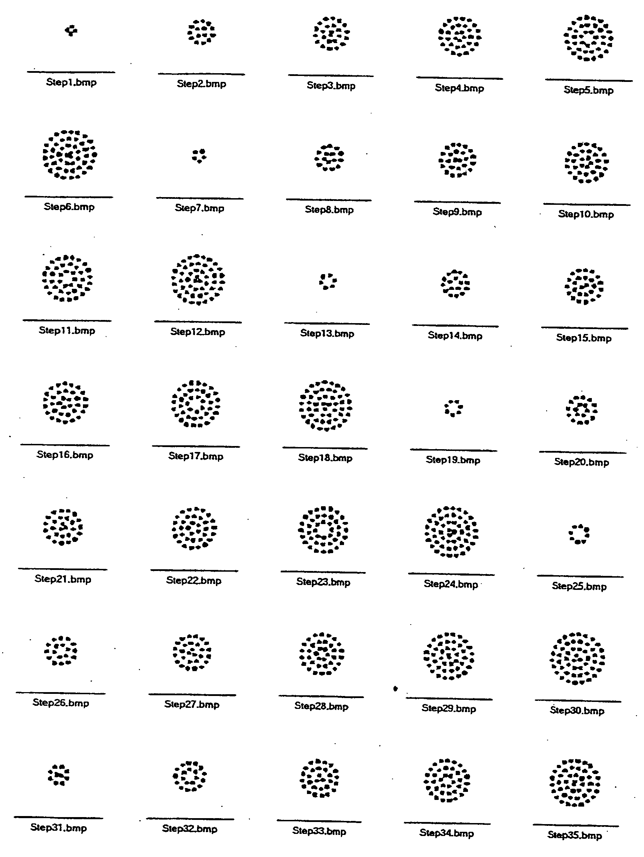

Zum besseren Verständnis des erfindungsgemäßen Verfahrens soll zunächst Figur 2, die beispielhaft die Abfolge erforderlicher Bearbeitungsschritte für eine Myopie Korrektur im Falle der Anwendung des sogenannten Small Spot Scanning nach dem Stand der Technik darstellt, erläutert werden. Gemäß Figur 2 sind die einzelnen Ablationsschichten in stetigen Reihen angeordnet, wie sie bei einer Behandlung von sphärischer Kurzsichtigkeit im Small Spot Scanning verwendet würden. Die einzelnen Punkte kennzeichnen die Mittelpunkte der einzelnen Laserablationen eines kreisförmigen Laserstrahles mit etwa 1-2 mm Durchmesser. Bei geeignetem Überlappungsfaktor und geeignetem, z.B. gaußförmigen Strahlprofil ergibt sich aus all den einzelnen Laserschüssen pro Schicht jeweils ein bzgl. der zu erzeugenden Korrektur irgendwie gearteter, jedoch möglichst refraktiv gleichmäßiger Gewebeabtrag innerhalb eines Areals. Bei der Area Ablation Methode entspräche ein solches Areal jeweils einem einzigen Laserpuls. Erst nach Abfolge aller, im Beispiel nach Fig. 2 vierundfünfzig Steps, von denen jeder einen Abtrag einer Schicht darstellt, ist die gewünschte Korrektur mit einem in der Mitte stärkeren Gewebeabtrag erreicht. Jeder Abbruch der Oberflächenformung vor Erreichen des letzten Bearbeitungsschrittes ist hier mit den oben beschriebenen Nachteilen verbunden.

Im Gegensatz dazu, steht das vorgeschlagene erfindungsgemäße Verfahren, welches anhand einer Abfolge erforderlicher Bearbeitungsschritte für eine Myopie Korrektur unter Einsatz des Small Spot Scanning, wie in Fig. 3 dargestellt, beschrieben werden soll. In Figur 3 ist die beim Verfahren verwendete Folge der einzelnen Ablationsschichten dargestellt. Dabei bildet jeweils eine gemäß der Erfindung zusammengefaßte Serie von Schichtabtragungen (vgl. Step1 bis 6; 7-12; 13-18; 19-24; 25-30; 31-36; 37- 42; 43-48; 49-54) jeweils eine eigene Teilkorrektur. Die genaue Anzahl der Schichten pro Teilkorrektur läßt sich in bestimmten Bereichen verändern. Zum einen sollte eine Mindestanzahl von Schichten nicht unterschritten werden damit eine theoretische Annäherung an eine sphärische Oberfläche überhaupt möglich ist. Zum anderen sollte der refraktive Effekt der Teilkorrektur so gering wie möglich gehalten werden, um bei einem Behandlungsabbruch entstehende Restunregelmäßigkeiten in einem vertretbar geringem refraktiven Rahmen zu halten.
Als sinnvoll erscheint eine Mindestzahl von 5 Schichten und eine bestimmbare Höchstzahl, die einer Korrektur von annähernd einer halben Dioptrie entspräche. Bei einer Ablationstiefe von 9 µm pro dpt für eine 5 mm Myopie Behandlung und einer typischen Schichtdicke von 0.3 µm wären dies bspw. 15 Schichten.
Unter Verwendung eines Lasers 1 mit gepulstem Laserausgangsstrahl, bspw. einem UV-Laser, wie Excimerlaser, Er:YAG-Laser oder gütegeschaltetem Laser für den vorgesehenen Verwendungszweck geeigneter Wellenlänge und Intensität bzw. Energie und der Verwendung von auf die Laserimpulsfolge abgestimmten Laserstrahlablenkeinheit 3, die durch ansteuerbare Kippspiegel o.ä. gebildet sein kann, wird im Bearbeitungsgebiet ein Setzen von Laserspots derart vorgenommen, daß in Abhängigkeit vom zu bearbeitenden, vom Sollwert abweichenden Linsenprofil (Myopie, Hyperopie, Astigmatismus, Cornea-Narben o.ä.) folgende Schritte ausgeführt werden:
a) in einem ersten vorgegebenen Gebiet werden mehrere Laserspots nacheinander, flächenmäßig möglichst gleichmäßig, jedoch zufällig verteilt gesetzt,
b) in einem gegenüber dem ersten Gebiet vergrößerten oder verkleinerten zweitem Gebiet, analog zum ersten Gebiet wiederum mehrere Einzellaserspots mit der gleichen Maßgabe wie unter a), jedoch zu den Einzellaserspots des ersten Gebietes versetzt und in gleicher oder veränderter Anzahl gesetzt werden,
c) der Vorgang des Laserspotsetzens in n Gebieten, wobei n zumindest fünf betragen soll, jeweils mit den Maßgaben nach a) und b) solange fortgesetzt wird, bis flächenmäßig das gesamte Bearbeitungsgebiet zu einem ersten Mal erfaßt und weitestgehend refraktiv gleichmäßig abgetragen ist,
d1) an dieser Bearbeitungsstufe ein ggf. erforderlicher Abbruch des Abtragungsverfahrens vorgenommen wird, wodurch wenigstens eine optische Teilkorrektur realisiert ist, oder
d2) die Bearbeitungsschritte nach a) bis c) wiederholt solange fortgeführt werden, bis ein gleichmäßiger, dem angestrebten Sollwert der Oberfläche entsprechender Abtrag des gesamten Bearbeitungsgebietes erreicht ist, wodurch ein refraktiver, insbesondere sphärischer Abtrag der zu bearbeitenden Oberfläche 4 erreicht wird.

Vergleicht man Fig. 3, ist zu erkennen, daß in einem ersten Schritt (Step1) zeitlich aufeinanderfolgend vier Laserspots entsprechend der Maßgabe a) gesetzt werden. Das heißt also nach dem Setzen des jeweils aktuellen Laserspots erfolgt vorzugsweise erst das jeweilige Setzen weiter von ihm entfernter Laserspots, wodurch eine lokale Überhitzung, infolge des Setzens mehrerer Spots in einem kleinen Areal, wirkungsvoll verhindert wird. Bevorzugt wird erst bei den jeweils folgenden Abtragungsschichten (Step2, ...,n) eine teilweise Überlappung vorheriger Abtragungsbereiche durch die Ansteuereinheit 5 zugelassen. In dem sich an Step1 anschließenden zweiten Bearbeitungsschritt (Step2) werden mehrere Laserspots in einem etwas vergrößerten Gebiet gesetzt, wobei die entsprechend Step1 mittig gesetzten vier Laserspots im Beispiel bei Step2 bereits versetzt zu den nach Step1 gesetzt werden. Betrachtet man im weiteren nur die im mittleren Areal nach Step1 gesetzten Laserspots (vgl Step3 bis 6), so ist zu erkennen, daß im weiteren auch die Anzahl der Laserspots und ihre Koordinaten verändert werden. Das gleiche gilt ebenso für die das mittige Areal umgebenden weiteren Areale. Die erzeugte erste Folge (Step1 bis 6) von Abtragungsschritten ergibt danach bereits eine erste optische Teilkorrektur, bei der gemäß der Maßgabe d1) ggf. ein erforderlicher Abbruch des Formgebungsverfahrens ohne nachteilige Wirkungen erfolgen könnte. Betrachtet man in Fig. 3 das beispielhaft erläuterte Verfahren bis zum letzten Bearbeitungsschritt in den einzelnen Blocksequenzen, so ist zu erkennen, daß durch eine ständige Veränderung der Anzahl und der Koordinaten der einzelnen Laserspots im Ergebnis ein gleichförmiger Materialabtrag, mit einem in den mittigen Arealen höheren Abtrag, da dort bei jedem Step Laserspots gesetzt werden, erzielt wird. Bei entsprechender Verfahrensführung kann somit im Beispiel eine exakte sphärische Linsenkorrektur durchgeführt werden.

Weiterhin liegt es im Rahmen der Erfindung, daß das Formgebungsverfahren bei Variation des Laserspotdurchmessers und/oder der Laserstrahlenergiedichte und/oder des Laserspotprofils während der Durchführung der Verfahrensschritte nach den Maßgaben a) bis c) durch eine entsprechende Ansteuerung des Lasers 1 und/oder der Strahlformungseinheit 2 zunächst wenigstens einmal mit ersten Parametern ausgeführt wird, woran anschließend die Verfahrensschritte nach a) bis c) mit veränderten Parametern solange wiederholt durchgeführt werden, bis ein, einer Teilkorrektur entsprechender Bearbeitungszustand nach der Maßgabe d1) oder eine vollständige Korrektur nach der Maßgabe d2) erreicht wird.
Es liegt im besonderen im Rahmen der Erfindung, Laserspots mit unterschiedlichen Spotdurchmessern zu verwenden. Vorteilhafeter Weise wird mindestens ein Bearbeitungszyklus a) bis c) mit einem großen Laserspotdurchmesser und weitere mit kleineren Laserspotdurchmessern durchgeführt. Die Kombination von Bearbeitungszyklen mit unterschiedlichen Laserspotdurchmessern sichert sowohl die Realisierung kurzer Bearbeitungszeiten, als auch einen Formgebungsabtrag entsprechend der gewünschten Parameter. Gelangt also bspw. während des Feinabtrags ein Laserstrahl mit einem Durchmesser von 1 mm zur Anwendung, kann vorher zumindest in solchen Gebieten, in denen ein größerer Abtrag erforderlich ist, wenigstens einmal ein Formgebungsabtrag entsprechend der Maßgaben nach a) bis c) mit einem größeren Laserstrahldurchmesser, bspw. mit 2 mm, durchgeführt werden.

In einer weiteren Ausführung läßt sich die Verkürzung der Bearbeitungszeit bei gleichzeitiger Sicherung der gewünschten Abtragung dadurch realisieren, daß zwischen Laser 1 und zu bearbeitender Oberfläche 4 ein die Energiedichte der Laserstrahlung beeinflussendes Element angeordnet ist. Ein solches Element kann z.B. ein mechanischer oder variabler optischer Abschwächer sein. Auch liegt es im Rahmen der Erfindung mindestens einen Bearbeitungszyklus a) bis c) mit hoher Energiedichte und weitere mit niederer Energiedichte durchzuführen.

Die Erfindung ist nicht auf die beschriebenen Ausführungsbeispiele begrenzt. So liegt es z.B. ebenso im Rahmen der Erfindung, daß einzelne Abtragungsgebiete vermittels der Strahlformungseinheit 2; 21 und/oder der Laserstrahlablenkeinheit 3 durch in sich geschlossene Ringbahnen oder spaltartige Abtragungszonen gebildet werden, wodurch sich durch das erfindungsgemäße Abtragungsverfahren im Grunde beliebige, vom sphärischen Sollwert abweichende Linsenkorrekturen vornehmen lassen.

Für die im Rahmen der Erfindung eingesetzten Laser kommen die für die PRK bzw. das LASIK-Verfahren vorgeschlagenen Laser in Betracht. Vorzugsweise ist der Laser ein Excimerlaser der Wellenlänge 193 nm oder ein Er:YAG-Laser der Wellenlänge 2940 nm. Bei Verwendung eines Excimerlasers beträgt die auf der zu bearbeitenden Oberfläche applizierte Energiedichte in einer erfindungsgemäßen Ausführung vorzugsweise 100 - 300 mJ/cm². Der Laserspot auf der zu bearbeitenden Oberfläche 4 ist weitgehend kreisrund und besitzt für mindestens eine Abtragungsschicht einen Laserspotdurchmesser von 3 mm. Weitere Schichten werden mit einem Laserspotdurchmesser von 1 mm abgetragen. Das Strahlprofil kann hier insbesondere eine gaußförmige Verteilung aufweisen, aber auch eine topfförmige Verteilung ist möglich. Es liegt im besonderen im Rahmen der Erfindung, den großflächigen Abtrag mit einer gaußförmigen Verteilung vorzunehmen und die Verteilung beim kleineren Durchmesser topfförmig zu wählen. Eine solche Kombination erlaubt es in besonderer Weise, sowohl einen kontinuierlichen Übergang in das unbehandelte Gebiet zu realisieren, als auch Unregelmäßigkeiten der Cornea, wie z.B. Narben zu behandeln.

Es liegt ausdrücklich im Rahmen der Erfindung, die beschriebene Methode zur ophthalmologisch exakten Formung von Kontaktlinsen anzuwenden.

### Bezugszeichenliste

- 1 -: Lasereinheit
- 2 -: Strahlformungseinheit
- 21 -: Blende
- 3 -: Laserstrahlablenkeinheit
- 4 -: zu bearbeitende Oberfläche
- 5 -: Ansteuereinheit
- 6 -: erste Eingabeeinheit
- 7 -: zweite Eingabeeinheit
- 8 -: Berechnungseinheit
- 9 -: Blockgenerators
- 10 -: Ablaufgenerator
- 11 -: Zentralrechnereinheit
- 12 -: Überwachungseinheit

## Patentansprüche

1. Verfahren zur Formgebung von Oberflächen, insbesondere von Linsen, vermittels Laserabtrag, unter Einsatz einer Vorrichtung nach den vorstehenden Ansprüchen, **dadurch gekennzeichnet, daß** in Abhängigkeit von der zu bearbeitenden, vom angestrebten Sollwert abweichenden Oberfläche
a) in einem ersten vorgegebenen Gebiet mehrere Laserspots nacheinander, flächenmäßig möglichst gleichmäßig, jedoch zufällig verteilt gesetzt werden,
b) in einem gegenüber dem ersten Gebiet vergrößerten oder verkleinerten zweitem Gebiet, analog zum ersten Gebiet wiederum mehrere Einzellaserspots mit der gleichen Maßgabe wie unter a), jedoch zu den Einzellaserspots des ersten Gebietes versetzt und in gleicher oder veränderter Anzahl gesetzt werden,
c) der Vorgang des Laserspotsetzens in n Gebieten, wobei n zumindest fünf betragen soll, jeweils mit den Maßgaben nach a) und b) solange fortgesetzt wird, bis flächenmäßig das gesamte Bearbeitungsgebiet zu einem ersten Mal erfaßt und weitestgehend refraktiv gleichmäßig abgetragen ist,
d1) an dieser Bearbeitungsstufe ein ggf. erforderlicher Abbruch des Abtragungsverfahrens vorgenommen wird, wodurch wenigstens eine optische Teilkorrektur realisiert ist oder
d2)die Bearbeitungsschritte nach a) bis c) wiederholt solange fortgeführt werden, bis ein gleichmäßiger, dem angestrebten Sollwert der Oberfläche entsprechender Abtrag des gesamten Bearbeitungsgebietes erreicht ist.

2. Verfahren nach Anspruch 1, **dadurch gekennzeichnet, daß** zeitlich aufeinanderfolgende Einzellaserspots voneinander deutlich so beabstandet gesetzt werden, daß die pro Laserspot erzeugten Abtragungsgebiete keine Überlappung aufweisen,

3. Verfahren nach Anspruch 1, **dadurch gekennzeichnet, daß** innerhalb eines Teilbearbeitungszyklus, bis flächenmäßig das gesamte Bearbeitungsgebiet zu einem ersten Mal erfaßt und weitestgehend refraktiv gleichmäßig abgetragen ist, vorgebbare Teilgebiete mehr als einmal einem Laserspotabtrag ausgesetzt werden.

4. Verfahren nach Anspruch 1, **dadurch gekennzeichnet, daß** das Formgebungsverfahren bei Variation des Laserspotdurchmessers und/oder der Laserstrahlenergiedichte und/oder des Laserspotprofils während der Durchführung der Verfahrensschritte nach a) bis c) durch eine entsprechende Ansteuerung des Lasers (1) und/oder der Strahlformungseinheit (2) zunächst wenigstens einmal mit ersten Parametern ausgeführt wird, woran anschließend die Verfahrensschritte nach a) bis c) mit veränderten Parametern solange wiederholt durchgeführt werden, bis ein Bearbeitungszustand nach dem Verfahrensschritt d1) oder d2) erreicht wird.

5. Verfahren nach Anspruch 1, **dadurch gekennzeichnet, daß** zumindest in solchen Gebieten, in denen ein größerer Abtrag erforderlich ist, wenigstens einmal die Bearbeitungsschritte a) bis c) mit einem größeren Laserstrahlspotdurchmesser durchgeführt werden, woran anschließend die Schritte a) bis c) solange mit einem kleineren Laserstrahldurchmesser wiederholt durchgeführt werden, bis ein Bearbeitungszustand nach d1) oder d2) erreicht ist.

6. Verfahren nach Anspruch 1 oder 3, **dadurch gekennzeichnet, daß** einzelne Abtragungsgebiete vermittels der Strahlformungseinheit (2; 21) und/oder der Laserstrahlablenkeinheit (3) durch in sich geschlossene Ringbahnen gebildet werden.

7. Verfahren nach Anspruch 1 oder 3, **dadurch gekennzeichnet, daß** einzelne Abtragungsgebiete vermittels der Strahlformungseinheit (2; 21) und/oder der Laserstrahlablenkeinheit (3) durch spaltartige Abtragungszonen gebildet werden.
